# EUROPEAN PATENT SPECIFICATION

(11) **EP 0 392 681 B1**
(45) Date of publication and mention of the grant of the patent: **12.01.1994**
(21) Application number: 90303014.6
(22) Date of filing: 21.03.1990
(51) Int. Cl.: C07D 477/00, A61K 31/00

(54) **Antibacterial 2-carbapenem derivatives**
Antibakteriell wirkende 2-Carbapenem-Derivate
Dérivés de 2-carbapénem à activité antibactérienne

(30) Priority: 28.03.1989 WO PCT/US89/01281
(43) Date of publication of application: 17.10.1990
(73) Proprietor: PFIZER INC., New York, N.Y. 10017 (US)
(72) Inventor: Vytautas, John Jasys, New London, Connecticut (US)
(74) Representative: Wood, David John

(56) References cited:
- EP-A- 0 223 397
- EP-A- 0 260 042
- WO-A-88/08845
- US-A- 4 619 924
- US-A- 4 757 066
- JOURNAL OF ANTIBIOTICS vol. 36, no. 8, 1983, pages 1034-1039, Tokio, JP; T. MYADERA et al.: "Synthesis and in vitro activity of a new carbapenem, RS-533"

## Description

The present invention is directed to antibacterial 5R, 6S-6-(1R-hydroxyethyl)-2-(3-thiolanyl)thio-2-carbapenem-3-carboxylic acids, as depicted by the formula (I) below; the pharmaceutically-acceptable salts and in vivo hydrolyzable esters thereof; and intermediates useful in the preparation of said compounds.

There are numerous reports in the literature concerning antibacterial 2-(alkylthio)-2-carbapenems and related compounds. See, for example, Andrus et al., J. Am. Chem. Soc., vol. 106, pp. 1808-1811, 1984; Afonso et al., ibid., vol. 104, pp. 6139-6140, 1982; DiNinno et al., Tetrahedron Letters, vol. 23, pp. 3535-3538 (1982); Ganguly et al., J. Antimicrobial Chemotherapy, vol. 9, suppl. C, pp. 1-5 (1982); Ghosez et al., Tetrahedron, vol. 39, pp. 2493-2503, 1983; Girijavallabhan et al., J. Antibiotics, vol. 39, pp. 1182-1190, 1986; Girijavallabhan et al., Tetrahedron Letters, vol. 24, pp. 3179-3182, 1979; Leanza et al., Tetrahedron, vol. 39, pp. 2505-2513, 1983; and Shih et al., Heterocycles, vol. 21, pp. 29-40, 1984; and Miyadera et al., J. Antibiotics, vol. 36, pp 1034-1039, 1983.

In addition, antibacterial 5R,6S-6-(1R-hydroxy-ethyl)-2-(cis-1-oxo-3-thiolanylthio)-2-penem-3-carboxylic acid and 5R,6S-6-(1R-hydroxyethyl)-2-(1,1-dioxo-3-thiolanylthio)-2-penem-3-carboxylic acid have been disclosed by Hamanaka, U.S. Patent 4,619,924. More recently, the preferred diastereoisomer, 5R,6S-6-(1R-hydroxyethyl)-2-(1R-oxo-3S-thiolanylthio)-2-penem-3-carboxylic acid, was identified by Volkmann in International Application No. PCT/US87/01114, designating inter alia the United States of America, published on November 17, 1988 as WO-88/08845.

EP-A-0 223 397 discloses some 2-(1-oxo-3-thiolanyl)-2-penem antibiotics. Certain 2-(1(Oxo-1-imino-1-thiacycloalkyl)-Thiopenem derivatives have been disclosed in EP-A-0 260 042.

It has now been discovered that carbapenems substituted in analogy to the above-noted penems of Hamanaka and Volkmann are especially valuable antibacterial agents. These novel compounds are of the formula 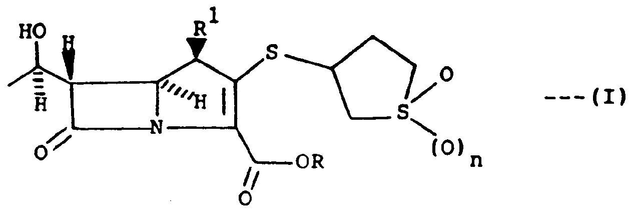
wherein n is 0 or 1; R is hydrogen or a radical forming an ester hydrolyzable under physiological conditions; and R¹ is hydrogen or methyl; including the pharmaceutically-acceptable cationic salts thereof when R is hydrogen.

Said pharmaceutically-acceptable cationic salts include, but are not limited to, those of sodium, potassium, calcium, N,N'-dibenzylethylenediamine, N-methylglucamine (meglumine) and diethanolamine. The preferred cationic salts are those of potassium and sodium.

The reference to esters which are hydrolyzable under physiological conditions refers to those esters frequently referred to as "pro-drugs." Such esters are now as well-known and common in the penicillin art as pharmaceutically-acceptable salts. Such esters are generally used to enhance oral absorption, but in any event are readily hydrolyzed in vivo to the parent acid. The more preferred ester forming radicals are those wherein R is:
(5-methyl-1,3-dioxol-2-on-4-yl)methyl;
1H-isobenzofuran-3-on-1-yl;
gamma-butyrolacton-4-yl;
-CHR²OCOR³; or
-CHR²OCOOR³;
wherein R² is hydrogen or methyl; and R³ is (C₁-C₆)alkyl. The most preferred radicals are pivaloyloxymethyl and 1-(ethoxycarbonyloxy)ethyl.

For ease of preparation, the preferred value of R¹ is hydrogen. When n is 0, it is preferred that the groups attached to the thiolane ring be cis to one another, most preferably in the 1R,3S-configuration, i.e.,

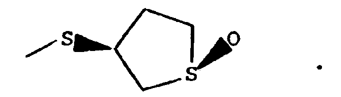

The present invention is also directed to intermediate compounds of the formula 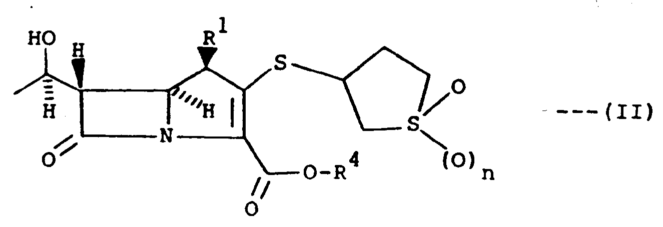
wherein n is 0 or 1; R¹ is hydrogen or methyl, and R⁴ is a conventional carboxylic acid protecting group such as benzyl, p-nitrobenzyl, or -CH₂CX=CH₂ where X is H or Cl. For their ease of preparation and the facile removal of the protecting group, the preferred compounds of the formula (II) are those wherein R⁴ is -CH₂CX=CH₂, most preferably with X as hydrogen.

The antibacterial compounds of the present invention, having the formula (I), are readily and conventionally prepared from the ketonic compound of the formula 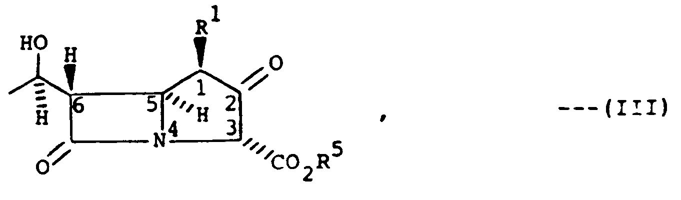
wherein R¹ is as defined above and R⁵ is a conventional carboxylic acid protecting group or a radical forming an ester hydrolyzable under physiological conditions; and a 3-thiolanyl mercaptan of the formula 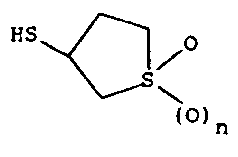
wherein n is as defined above. For example, the ketone (III) is initially reacted with substantially one molar equivalent of a reagent such as diphenyl chlorophosphate, (C₆H₅O)₂P(O)Cl, in the presence of substantially one molar equivalent of a hindered tertiary amine such as di(isopropyl)ethylamine to form the enol phosphate ester of the formula 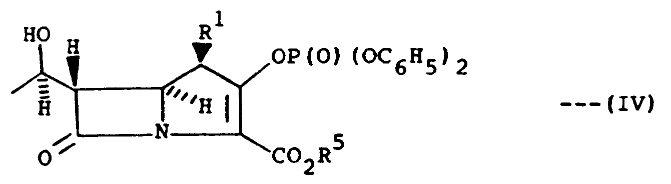
Generally without isolation, with an additional molar equivalent of the amine present, the phosphate (IV) is then reacted directly with substantially one molar equivalent of the appropriate 3-thiolanyl mercaptan, thus forming the intermediate compound of the formula (II) when R⁵ is a carboxylic acid protecting group such as CH₂CX=CH₂, or finished prodrug ester product of the formula (I) when R⁵ is a radical forming an ester hydrolyzable under physiological conditions. This reaction sequence is generally carried out in a reaction-inert solvent such as acetonitrile. While temperature is not particularly critical, it is preferred to operate in the range of about -20° to 30°C., conveniently at ice bath temperature (0-5° C.).

As used herein, the expression "reaction-inert solvent" refers to a solvent which does not interact with starting materials, reagents, intermediates or products in a manner which adversely affects the yield of the desired product.

When the product is an allyl or 2-chloroallyl ester of the formula (II), the ester is hydrolyzed to produce the desired penem of the formula (I), above, in the form of the acid or its pharmaceutically-acceptable cationic salt. Anhydrous conditions are generally employed to avoid any possible degradation of the betalactam. Preferred conditions employ 1 to 1.1 molar equivalents of an alkali metal salt of a lipophilic carboxylic acid (e.g., sodium 2-ethylhexanoate) in an anhydrous reaction-inert solvent (e.g., methylene chloride and/or ethyl acetate) in the presence of catalytic amounts of triphenylphosphine and tetrakis(triphenylphosphine)palladium (e.g., about 0.15 molar equivalents of the former and about 0.075 molar equivalents of the latter). This reaction is generally carried out under an inert atmosphere and protected from light. Although temperature is not critical, the reaction is conveniently carried out at ambient temperature. With these reagents, the compound of the formula (I) is usually initially isolated in the form of its alkali metal (e.g., sodium) salt. If desired, the salt is converted to the free acid form, during or after isolation, by standard methods, e.g., acidification of an aqueous solution of the salt, with extraction of the free acid into a water immiscible organic solvent. Alternative carboxylic acid protecting groups are removed by conventional means, e.g., benzyl and p-nitrobenzyl groups by hydrogenation over a noble metal catalyst such as Pd/C.

Other pharmaceutically-acceptable cationic salts of the present invention are also readily prepared by standard methods. For example, an equivalent of the corresponding cationic hydroxide, carbonate or bicarbonate or of an amine, is combined with the carboxylic acid in an organic or aqueous solvent, preferably at reduced temperature (e.g., 0-5° C.), with vigorous agitation and slow addition of the base. The salt is isolated by concentration and/or the addition of a non-solvent.

The compounds of the formula (I) wherein R represents an in vivo hydrolyzable ester are alternatively prepared from the corresponding free acids or cationic salts according to known methods, readily identified by those skilled in the penicillin art (see for example, U.S. Patents 3,951,954; 4,234,579; 4,287,181; 4,342,693; 4,452,796; 4,348,264; 4,416,891; and 4,457,924). In the present instance, the preferred precursors are generally in the form of a salt, preferably the tetrabutylammonium salt, which is reacted with the appropriate ester forming reagent, e.g., chloromethyl pivalate or 1-chloroethyl ethyl carbonate.

Concerning other starting materials required for the process of the present invention, the ketones of the formula (III) are readily available by literature methods (for example, see the carbapenem references cited above), and by means of preparative methods of the type detailed below. The required mercaptans are available according to above cited Hamanaka and Volkmann.

The in vitro activity of the compounds of the formula (I) is determined by measuring the minimum inhibitory concentration (MIC) of the free acids or cationic salts in mcg/ml against a variety of micro-organisms. The procedure which is followed is the one recommended by the International Collaborative Study on Antibiotic Sensitivity Testing (Ericcson and Sherris, Acta. Pathologica et Microbiologia Scandinav, Supp. 217, Section B: 64-68 [1971]), and employs brain heart infusion (BHI) agar and the inocula replicating device. Overnight growth tubes are diluted 100 fold for use as the standard inoculum (20,000-10,000 cells in approximately 0.002 ml are placed on the agar surface; 20 ml of BHI agar/dish). Twelve 2 fold dilutions of the test compound are generally employed, with initial concentration of the test drug being 100-200 mcg/ml. Single colonies are disregarded when reading plates after 18 hours at 37° C. The susceptibility (MIC) of the test organism is accepted as the lowest concentration of compound capable of producing complete inhibition of growth as judged by the naked eye.

The in vivo activity of the compounds of the formula (I) can be determined by conventional animal protection studies, usually carried out in mice. In this test groups of mice are usually infected intraperitoneally with multiple lethal doses of a microorganism. Each group of mice is dosed, usually orally or subcutaneously, with a particular dose of the antibacterial compound. Such in vivo results are generally reported in the form of a PD₅₀ value in mg/Kg, i.e., the dose of drug in mg/Kg which will protect 50% of mice from the infecting microorganism. This value is conveniently determined graphically, e.g., by plotting dose against % protection.

The present antibacterial compounds find primary use in animals, including man, in the systemic treatment of infections due to susceptible microorganisms. They are dosed at a level of 2.5-100 mg/kg per day, preferably 5-50 mg/kg/day, in single or divided doses. Variation in dosage will be made depending upon the animal and upon the particular susceptibility of the microorganism. These compounds are dosed orally or parenterally, the preferred route being oral, particularly when the antibiotic is in the form of a prodrug ester, as defined above. The susceptibility of microorganisms isolated in the field is routinely tested in bacteriology laboratories by the well-known disc-plate method. Compound (I) is generally the compound of choice when it shows a relatively large zone of inhibition against the bacteria causing the infection to be treated.

Preparation of optimal dosage forms will be by methods well known in the pharmaceutical art. For oral administration, the compounds are formulated alone or in combination with pharmaceutical carriers such as inert solid diluents, aqueous solutions or various nontoxic organic solvents in such dosage forms as gelatin capsules, tablets, powders, lozenges, syrups and the like. Such carriers include water, ethanol, benzyl alcohol; glycerin, propylene glycol, vegetable oils, lactose, starches, talc, gelatins, gums and other well known carriers. The parenteral dosage forms required for the above systemic use are dissolved or suspended in a pharmaceutically-acceptable carrier such as water, saline, sesame oil and the like. Agents which improve the suspendability and dispersion qualities can also be added.

Based on nothing more than their excellent in vitro activity, the present antibacterial compounds, particularly in free acid or salt form, also find use in the topical treatment of superficial infections in animals, including man, caused by susceptible microorganisms, the compound (I) is formulated by methods well known in the pharmacist's art into lotions, ointments, creams, salves, gels, or the like at concentrations in the range 5-200 mg/cc of the dosage form, preferably in the range 10-100 mg/cc. The dosage form is applied at the site of infection ad libitum, generally at least once a day.

The present invention is illustrated by the following examples.

### EXAMPLE 1

### Allyl (5R,6S)-6-(1R-Hydroxyethyl)-2-(1,1-dioxo-3-thiolanylthio)-2-carbapenem-3-carboxylate (II, n = 1, R⁴= CH₂CH=CH₂)

Under N₂, allyl (3R,5R,6S)-6-(1R-hydroxyethyl)-2-oxocarbapenam (0.109 g, 0.43 mmol) was dissolved in ml of CH₃CN and cooled to 0-5° C. Diphenyl chlorophosphate (0.114 g, 0.43 mmol) and then diisopropylethylamine (0.075 ml, 0.43 mmol) were added and the mixture stirred for 30 minutes at 0-5° C. to form a solution of the intermediate enol phosphate ester of the above formula (IV) wherein R⁵ is allyl. 3-Thiolanyl mercaptan 1,1-dioxide (0.065 g, 0.43 mmol) and a second equivalent of the amine (0.075 ml, 0.43 mmol) were added. After stirring for an additional 1 hour at 0-5° C., the reaction mixture was poured into 75 ml of ethyl acetate, extracted in sequence with 1 x 15 ml H₂O, 2 x 15 ml saturated NaHCO₃, 1 x 15 ml H₂O and 1 x 15 ml brine, dried (Na₂SO₄), stripped of solvent and the residue chromatographed on silica gel using ethyl acetate as eluant to yield 38 mg of present title product, an approximately 1:1 mixture of 3R and 3S-thiolanyl side chain diastereoisomers; TLC Rf 0.2 (ethyl acetate); ¹H-NMR (CDCl₃)delta(ppm) 1.32 (d, 3H), 2.04 (bs, 1H), 2.14-2.26 (m, 1H) , 2.57-2.67 (m, 1H) , 2.97-3.36 (m, 6H) , 3.41-3.51 (m, 1H), 3.76-3.88 (m, 1H) , 4.17-4.28 (m, 2H) , 4.79 (dd, 2H) , 5.24 (d, 1H) , 5.41 (d, 1H), 5.86-5.99 (m, 1H); HRMS 387.0765, calcd. 387.0810.

To avoid product loss to the aqueous phases, it is preferable to directly chromatograph the reaction mixture, as in Example 3 below.

### EXAMPLE 2

### Sodium (5R,6S)-6-(1R-Hydroxyethyl)-2-(1,1-dioxo-3-thiolanylthio)-2-carbapenem-3-carboxylate (I, n = 1, R = H as the sodium salt)

Under N₂ and in a flask wrapped with aluminum foil, title product of the preceding Example (19 mg, 0.05 mmol) was dissolved in 0.5 ml CH₂Cl₂. Triphenylphosphine (2.6 mg) and sodium 2-ethylhexanoate (8.3 mg in 0.43 ml of ethyl acetate) were added and the mixture protected from light. Finally tetrakis(triphenylphosphine)palladium (5 mg) was added and the mixture stirred for 45 minutes. Present title product (14.4 mg) was recovered by filtration with ethyl acetate wash; ¹H-NMR (D₂O)delta(ppm) 1.27 (d, 3H), 2.2-2.3 (m, 1H), 2.65-2.69 (m, 1H), 3.11-3.30 (m, 4H), 3.4-3.46 (m, 2H), 3.65-3.74 (m, 1H), 4.04-4.23 (m, 3H); MS 370 (M⁺); IR(KBr) includes 1754 cm⁻¹.

### EXAMPLE 3

### Allyl (5R,6S)-6-(1R-Hydroxyethyl)-2-(1R-oxo-3S-thiolanylthio)-2-carbapenem-3-carboxylate (II, n = 0, R⁴= CH₂CH=CH₂)

With stirring under N₂, 3S-(acetylthio)thiolane 1R-oxide (0.712 g, 4 mmol; Volkmann, WO 88/08845) was dissolved in 2.5 ml H₂O and cooled to -5° C. NaOH (0.32 g, 8 mmol) was added, and the mixture warmed to 0-5° C., stirred for 30 minutes, acidified with 0.8 ml 12N HCl at 5-10° C., saturated with Na₂SO₄ at ambient temperature and extracted 4 x 7 ml CH₂Cl₂. The organic extracts were combined, dried (Na₂SO₄) and filtered to yield a solution of 3S-thiolanyl mercaptan 1R-oxide used without isolation directly below.

In a separate flask, allyl (3R,5R,6S)-6-(1R-hydroxy-ethyl)-2-oxocarbapenam (0.506 g, 2 mmol) was converted to a solution of the phosphate ester intermediate and then reacted with said mercaptan solution according to the methods of Example 1. The entire reaction mixture was chromatographed directly on silica gel using 9:1 ethyl acetate:CH₃OH as eluant. The initial product residue was rechromatographed using acetone as eluant to yield 0.428 g of present, purified title product as a foam; TLC Rf 0.1 (4:1 ethyl acetate:CH₃OH), 0.25 (acetone) ; ¹H-NMR (CDCl₃)delta(ppm) 1.3 (d, 3H), 2.59-2.79 (m, 4H), 3.05-3.26 (m, 4H), 3.52-3.58 (m, 1H), 3.74-3.82 (m, 1H), 4.15-4.25 (m, 2H), 4.73 (dd, 2H), 5.27 (d, 1H), 5.40 (d, 1H), 5.86-5.97 (m, 1H).

### EXAMPLE 4

### Sodium (5R,6S)-6-(1R-Hydroxyethyl)-2-(1R-oxo-3S-thiolanylthio)-2-carbapenem-3-carboxylate n = 0, R⁴ = H, sodium salt)

By the method of Example 2, title product of the preceding Example (0.34 g, 0.92 mmol) was converted to 0.28 g of present title product.

Further purification was achieved dissolving 0.27 g of this product in 40 ml of water, treating with 0.4 g of activated carbon at 0-5° C. for 20 minutes, filtering, extracting the filtrate 2 x 25 ml ethyl acetate, and freeze drying the aqueous layer to yield 0.20 g of present, purified title product; ¹H-NMR (D₂O)delta(ppm) 1.26 (d, 3H), 2.41-2.48 (m, 1H), 2.69-2.75 (m, 1H), 2.85-2.90 (m, 1H), 2.91-2.98 (m, 1H), 3.15-3.39 (m, 3H) , 3.39 (dd, 1H) , 3.82-3.92 (m, 2H), 4.18-4.23 (m, 2H); MS 354 (M⁺); IR(KBr) includes 1761 cm⁻¹.

### PREPARATION 1

### Allyl 3-Pyrrolidino-2-butenoate

Allyl acetoacetate (68.4 ml, 0.5 mol) was added to pyrrolidine (41.6 ml, 0.5 mol) in 150 ml of toluene. An exotherm was noted. The mixture was heated at reflux for 3 hours, cooled, and stripped of toluene to yield 45 g of title product as a pale yellow oil.

### PREPARATION 2

### (3S,4R)-4-[3-(Allyloxycarbonyl)-2-pyrrolidino-2-propenyl]-3-[1R-1-(dimethyl-t-butylsilyloxy)ethyl]-2-azetidinone

Under N₂, title product of the preceding Preparation (9.75 g, 0.05 mol) in 100 ml dry tetrahydrofuran was cooled to -60° C. Maintaining a temperature below -50° C., butyllithium (31 ml of 1.6M in hexanes, 0.051 mol) was added, and the mixture stirred 20 minutes at -60° C. and 30 minutes at 0° C., and then recooled to -60° C. Diethylaluminum chloride (50 ml) was added and stirring continued at -60° C. for 20 minutes, at which time 3R,4R-4-acetoxy-3-[1R-1-(dimethyl-t-butylsilyloxy)-ethyl]-2-azetidinone (5.74 g, 0.02 mol; Leanza et al., Tetrahedron, vol. 39, pp. 2505-2513, 1983) in 25 ml dry tetrahydrofuran was added, and the mixture further stirred 20 minutes at -60° C. and 30 minutes at 0-5° C. The reaction mixture was then poured into 400 ml of ice and water and 400 ml of ethyl acetate, filtered to remove insoluble byproducts and the layers separated. The aqueous layer was extracted with 400 ml fresh ethyl acetate and the organic layers were combined, backwashed 4 x 200 ml H₂O and then 1 x 200 ml brine, dried (Na₂SO₄) and stripped of solvent. The residue was chromatographed on silica gel using 3:1 hexane:acetone as eluant to yield 4.59 g of present title product; TLC Rf 0.2 (3:1 hexane:acetone).

### PREPARATION 3

### (3S,4R)-4-[3-(Allyloxycarbonyl)-2-oxopropyl]-3-[1R-1-(dimethyl-t-butylsilyloxy)ethyl]-2-azetidinone

Title product of the preceding Preparation (4.38 g, 0.0104 mol) was combined with 75 ml tetrahydrofuran, 4 ml acetic acid and 1 ml H₂O. After stirring for 24 hours, the mixture was diluted with 250 ml ethyl acetate, washed in sequence with 4 x 50 ml H₂O, 2 x 50 ml saturated NaHCO₃, 1 x 50 ml H₂O and 1 x 50 ml brine, dried (Na₂SO₄), stripped of solvent, twice restripped from 25 ml of CH₂Cl₂ and pumped dry in high vacuum to yield 3.8 g of present title product; TLC Rf 0.3 (1:1 hexane:ethyl acetate).

### PREPARATION 4

### (3S,4R)-4-[3-(Allyloxycarbonyl)-2-oxopropyl]-3-[1R-1-hydroxy-ethyl]-2-azetidinone

Title product of the preceding Preparation (0.82 g, 2.2 mmol) was dissolved in 15 ml CH₃OH and cooled to 0-5° C. with stirring. 6N HCl (1.5 ml, 9 mmol) was added and the mixture allowed to warm to room temperature and, after stirring for 2 hours, poured into 25 ml of water and neutralized (pH 7) with 2% NaHCO₃. The mixture was then saturated with NaCl and extracted 4 x 30 ml CH₂Cl₂. The extracts were combined, dried (Na₂SO₄), stripped of solvent, and the residue chromatographed on silica gel using 19:1 ethyl acetate:CH₃OH as eluant to yield 0.38 g of present title product; TLC Rf 0.2 (ethyl acetate).

### PREPARATION 5

### (3S,4R)-4-[3-(Allyloxycarbonyl)-3-diazo-2-oxopropyl]-3-[1R-hydroxyethyl-2-azetidinone

By the method of Ratcliffe et al., Tetrahedron Letters, vol. 21, pp. 31-34, title product of the preceding Preparation (0.38 g, 1.5 mmol) in 15 ml of CH₃CN at 0-5° C. under N₂ was reacted with p-carboxy-benzenesulfonylazide (0.34 g, 1.5 mmol) in the presence of triethylamine (0.41 ml, 3 mmol). The reaction mixture was warmed to room temperature, stirred for 30 minutes, filtered, and the filtrate stripped of solvent. The residue was pumped dry to yield present title product; TLC Rf 0.33 (ethyl acetate); all of which was used in the next step.

### PREPARATION 6

### Allyl (3R,5R,6S)-6-(1R-Hydroxy-ethyl)-2-oxocarbapenam (III, R⁵= CH₂CH=CH₂)

Under N₂, the entire product of the preceding Preparation (1.5 mmol) was taken into 50 ml of C₆H₆. Rh₂(CH₃CO₂)₄ (25 mg) was added and the mixture heated to reflux for 12 minutes, then cooled, filtered on a millipore filter and the filtrate stripped of solvent. The semisolid residue was triturated and restripped to a second residue which was chromatographed on silica gel using 19:1 ethyl acetate:CH₃OH as eluant to yield 0.285 g of present title product as an oil. Trituration with ether gave 0.119 g of crystalline title product; TLC Rf 0.6 (ethyl acetate).

This Preparation was repeated using only 4.2 ml of C₆H₆ with 12.6 ml of ethyl acetate as cosolvent to yield 0.142 g of present title product from the ether trituration, and an additional 0.054 g by concentration of the ether mother liquor.

## Claims (Claims for the following Contracting State(s): AT, BE, CH, DE, DK, FR, GB, IT, LI, LU, NL, SE)

1. A compound having the formula 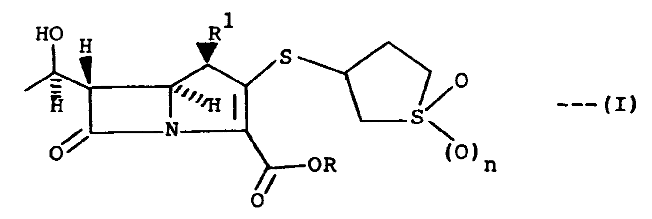 wherein n is 0 or 1; R is hydrogen or a radical forming an ester hydrolyzable under physiological conditions; and R¹ is hydrogen or methyl; or a pharmaceutically-acceptable cationic salt thereof when R is hydrogen.

2. A compound of claim 1 wherein R¹ is hydrogen.

3. A compound of claim 2 wherein R is hydrogen.

4. The compound of claim 3 wherein n is 1.

5. A compound of claim 3 wherein n is 0.

6. A compound of claim 5 wherein the groups attached to the thiolane ring are cis to one another.

7. The compound of claim 6 wherein configurations on the thiolane ring are 1R,3S, i.e.,

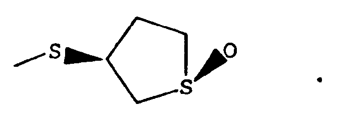

8. A compound of claim 2 wherein R is a radical group forming an ester hydrolyzable under physiological conditions.

9. A compound as claimed in claim 1 or 2 wherein R is (5-methyl-1,3-dioxol-2-on-4-yl)methyl, 1-H-isobenzofuran-3-on-1-yl, gamma-butyrolacton-4-yl, -CHR²OCOR³ or -CHR²OCOOR³ where R² is hydrogen or methyl and R³ is C₁-C₆ alkyl.

10. A compound having the formula 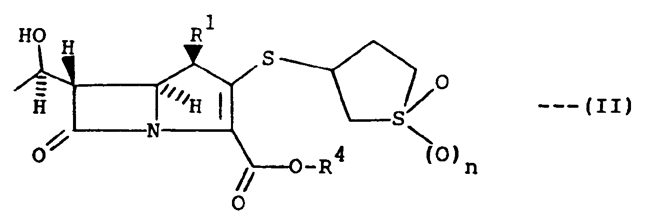 wherein n is 0 or 1; R¹ is hydrogen or methyl, and R⁴ is a conventional carboxylic acid protecting group.

11. A compound as claimed in claim 10 wherein R⁴ is benzyl, p-nitrobenzyl or -CH₂CX=CH₂ where X is H or Cl.

12. A compound of claim 11 wherein R¹ is hydrogen and R⁴ is -CH₂CX=CH₂ ,where X is hydrogen or chloro.

13. A pharmaceutical composition comprising a compound or salt thereof as claimed in any one of claims 1 to 9 and a pharmaceutically acceptable diluent or carrier.

14. A compound or salt thereof as claimed in any one of claims 1 to 9 for use as a medicament.

15. The use of a compound or salt thereof as claimed in any one of claims 1 to 9 for the manufacture of an antibacterial agent.

## Claims (Claims for the following Contracting State(s): ES)

1. A process for the preparation of a compound having the formula 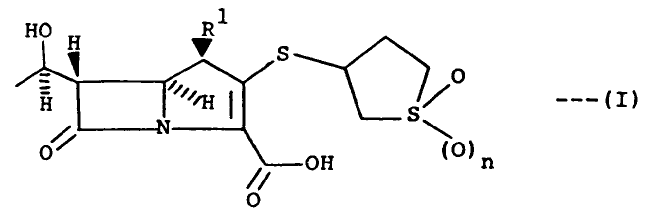 wherein n is 0 or 1; and R¹ is hydrogen or methyl; or a pharmaceutically-acceptable cationic salt thereof when R is hydrogen; which comprises conventional deprotection of a compound having the formula 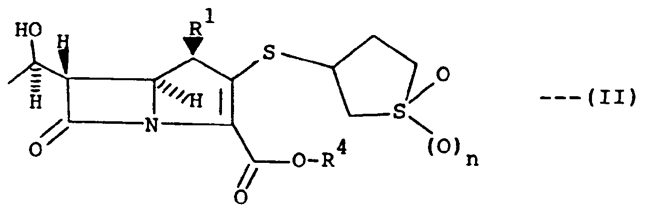 wherein n is 0 or 1, R¹ is hydrogen or methyl, and R⁴ is a conventional carboxylic acid protecting group.

2. A process of claim 1 wherein R¹ is hydrogen.

3. The process of claim 2 wherein n is 1.

4. A process of claim 2 wherein n is 0.

5. A process of claim 4 wherein the groups attached to the thiolane ring are cis to one another.

6. The process of claim 5 wherein configurations on the thiolane ring are 1R,3S, i.e.,

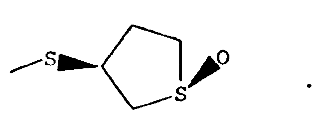

7. A process of claim 1 or 2 wherein R⁴ is -CH₂CX=CH₂ in which X is hydrogen or chloro.

## Claims (Claims for the following Contracting State(s): GR)

1. A process for the preparation of a compound having the formula 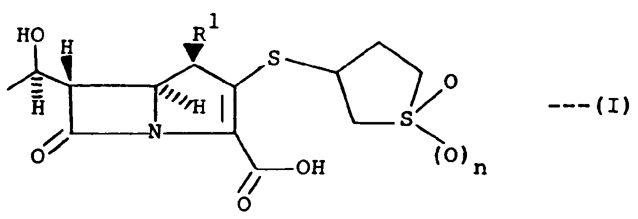 wherein n is 0 or 1; R¹ is hydrogen or methyl, or a pharmaceutically-acceptable cationic salt thereof when R is hydrogen; which comprises conventional deprotection of a compound having the formula 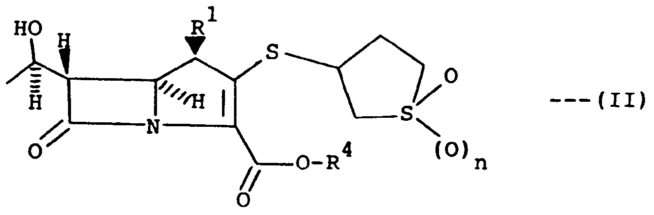 wherein n is 0 or 1, and R¹ is hydrogen or methyl, and R⁴ is a conventional carboxylic acid protecting group.

2. A process of claim 1 wherein R¹ is hydrogen.

3. The process of claim 2 wherein n is 1.

4. A process of claim 2 wherein n is 0.

5. A process of claim 4 wherein the groups attached to the thiolane ring are cis to one another.

6. The process of claim 5 wherein configurations on the thiolane ring are 1R,3S, i.e.,

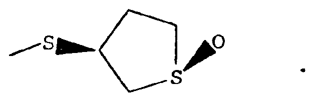

7. A process of claim 1 or 2 wherein R⁴ is -CH₂CX=CH₂ in which X is hydrogen or chloro.

8. A compound having the formula 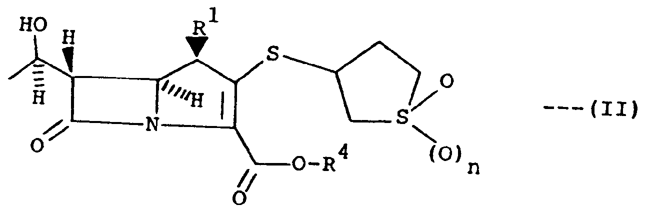 wherein n is 0 or 1, R¹ is hydrogen or methyl, and R⁴ is a conventional carboxylic acid protecting group.

9. A compound of claim 8 wherein R¹ is hydrogen and R⁴ is -CH₂CX=CH₂ where X is hydrogen or chloro.

## Patentansprüche (Patentansprüche für folgende(n) Vertragsstaat(en): AT, BE, CH, DE, DK, FR, GB, IT, LI, LU, NL, SE)

1. Verbindung mit der Formel 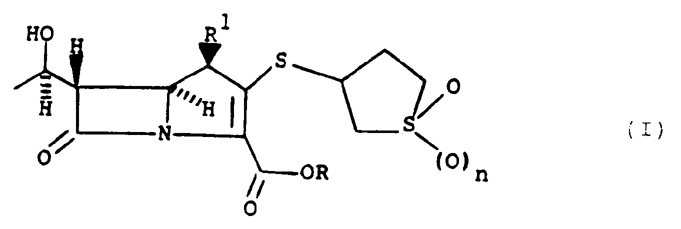 worin n 0 oder 1 ist, R Wasserstoff oder eine Gruppe ist, die einen unter physiologischen Bedingungen hydrolysierbarn Ester bildet, und R¹ Wasserstoff oder Methyl ist, oder ein pharmazeutisch annehmbares kationisches Salz davon, wenn R Wasserstoff ist.

2. Verbindung nach Anspruch 1, worin R¹ Wasserstoff ist.

3. Verbindung nach Anspruch 2, worin R Wasserstoff ist.

4. Verbindung nach Anspruch 3, worin n 1 ist.

5. Verbindung nach Anspruch 3, worin n 0 ist.

6. Verbindung nach Anspruch 5, worin die an den Thiolanring gebundenen Gruppen zueinander in cis-Position angeordnet sind.

7. Verbindung nach Anspruch 6, worin die Konfigurationen auf den Thiolanring 1R,2S sind, d.h.

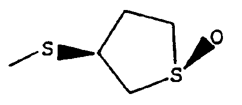

8. Verbindung nach Anspruch 2, worin R eine Gruppe ist, die einen unter physiologischen Bedingungen hydrolysierbaren Ester bildet.

9. Verfahren nach Anspruch 1 oder 2, worin R (5-Methyl-1,3-dioxol-2-on-4-yl)methyl, 1H-Isobenzofuran-3-on-1-yl, gamma-Butyrolacton-4-yl, -CHR²OCOR³ oder -CHR²OCOOR³ ist, wobei R² Wasserstoff oder Methyl ist und R³ C₁-C₆-Alkyl ist.

10. Verbindung mit der Formel 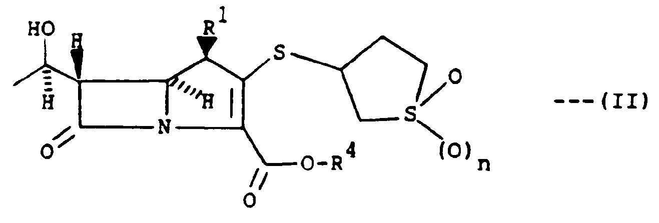 worin n 0 oder 1 ist, R¹ Wasserstoff oder Methyl ist und R⁴ eine übliche Carbonsäure-Schutzgruppe ist.

11. Verbindung nach Anspruch 10, worin R⁴ Benzyl, p-Nitrobenzyl oder -CH₂-CX=CH₂ ist, wobei X H oder Cl ist.

12. Verbindung nach Anspruch 11, worin R1 Wasserstoff ist und R⁴ -CH₂CX=CH₂ ist, wobei X Wasserstoff oder Chlor ist.

13. Pharmazeutische Zusammensetzung, die eine Verbindung oder deren Salz nach irgendeinem der Ansprüche 1 bis 9 und ein pharmazeutisch annehmbares Verdünnungsmittel oder einen solchen Träger umfaßt.

14. Verbindung oder deren Salz nach irgendeinem der Ansprüche 1 bis 9 zur Verwendung als Arzneimittel.

15. Verwendung einer Verbindung oder des Salzes davon nach irgendeinem der Ansprüche 1 bis 9 zur Herstellung eines antibakteriellen Mittels.

## Patentansprüche (Patentansprüche für folgende(n) Vertragsstaat(en): ES)

1. Verfahren zum Herstellen einer Verbindung mit der Formel 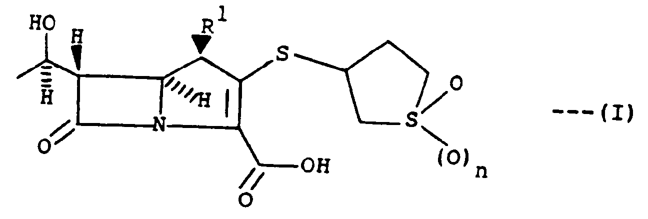 worin n 0 oder 1 ist und R¹ Wasserstoff oder Methyl ist, oder eines pharmazeutisch annehmbaren kationischen Salzes hiervon, sofern R Wasserstoff ist, das die übliche Schutzgruppenentfernung von einer Verbindung mit der Formel 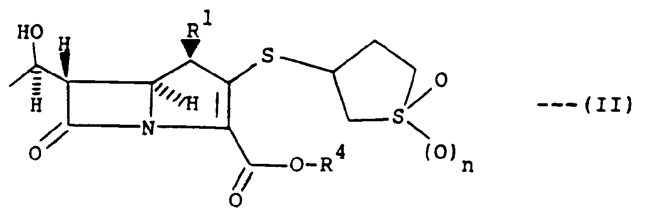 worin n 0 oder 1 ist, R¹ Wasserstoff oder Methyl ist und R⁴ eine übliche Carbonsäure-Schutzgruppe ist, umfaßt.

2. Verfahren nach Anspruch 1, worin R¹ Wasserstoff ist.

3. Verfahren nach Anspruch 2, worin n 1 ist.

4. Verfahren nach Anspruch 2, worin n 0 ist.

5. Verfahren nach Anspruch 4, worin die an den Thiolanring gebundenen Gruppen zueinander in cis-Position angeordnet sind.

6. Verfahren nach Anspruch 5, worin die Konfigurationen auf den Thiolanring 1R,2S sind, d.h.

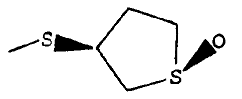

7. Verfahren nach Anspruch 1 oder 2, worin R⁴ -CH₂CX=CH₂ ist, in welchem X Wasserstoff oder Chlor ist.

## Patentansprüche (Patentansprüche für folgende(n) Vertragsstaat(en): GR)

1. Verfahren zum Herstellen einer Verbindung mit der Formel 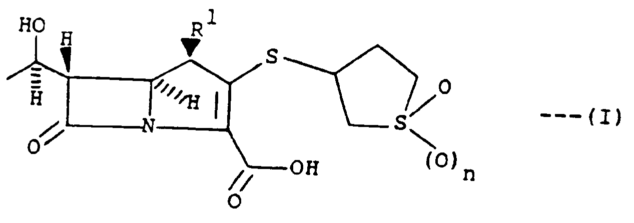 worin n 0 oder 1 ist und R¹ Wasserstoff oder Methyl ist, oder eines pharmazeutisch annehmbaren kationischen Salzes hiervon, sofern R Wasserstoff ist, das die übliche Schutzgruppenentfernung von einer Verbindung mit der Formel 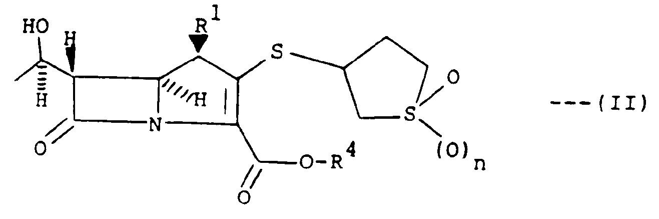 worin n 0 oder 1 ist, R¹ Wasserstoff oder Methyl ist und R⁴ eine übliche Carbonsäure-Schutzgruppe ist, umfaßt.

2. Verfahren nach Anspruch 1, worin R¹ Wasserstoff ist.

3. Verfahren nach Anspruch 2, worin n 1 ist.

4. Verfahren nach Anspruch 2, worin n 0 ist.

5. Verfahren nach Anspruch 4, worin die an den Thiolanring gebundenen Gruppen zueinander in cis-Position angeordnet sind.

6. Verfahren nach Anspruch 5, worin die Konfigurationen auf den Thiolanring 1R,2S sind, d.h.

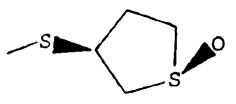

7. Verfahren nach Anspruch 1 oder 2, worin R⁴ -CH₂CX=CH₂ ist, in welchem X Wasserstoff oder Chlor ist.

8. Verbindung mit der Formel 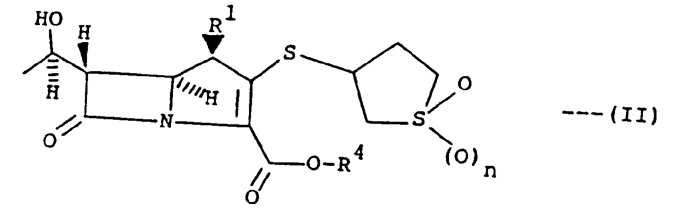 worin n 0 oder 1 ist, R¹ Wasserstoff oder Methyl ist und R⁴ eine übliche Carbonsäure-Schutzgruppe ist.

9. Verbindung nach Anspruch 8, worin R¹ Wasserstoff ist und R⁴ -CH₂CX=CH₂ ist, wobei X Wasserstoff oder Chlor ist.

## Revendications (Revendications pour l'(les) Etat(s) contractant(s) suivant(s): AT, BE, CH, DE, DK, FR, GB, IT, LI, LU, NL, SE)

1. Composé répondant à la formule 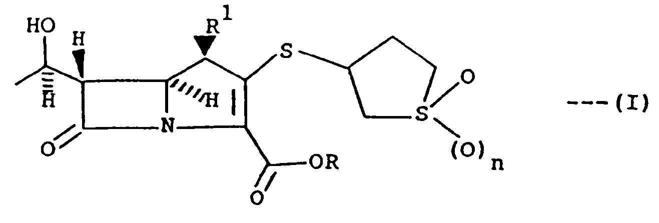 dans laquelle n est égal à 0 ou 1 ; R représente l'hydrogène ou un radical formant un ester hydrolysable dans les conditions physiologiques ; et R¹ représente l'hydrogène ou un groupe méthyle ; ou un de ses sels cationiques pharmaceutiquement acceptables lorsque R représente l'hydrogène.

2. Composé suivant la revendication 1, dans lequel R¹ représente l'hydrogène.

3. Composé suivant la revendication 2, dans lequel R représente l'hydrogène.

4. Composé suivant la revendication 3, dans lequel n est égal à 1.

5. Composé suivant la revendication 3, dans lequel n est égal à 0.

6. Composé suivant la revendication 5, dans lequel les groupes fixés au noyau thiolane sont en position cis l'un par rapport à l'autre.

7. Composé suivant la revendication 6, dans lequel les configurations sur le noyau thiolane sont les configurations 1R,3S, c'est-à-dire

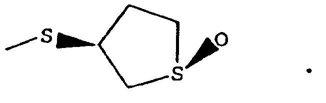

8. Composé suivant la revendication 2, dans lequel R représente un radical formant un ester hydrolysable dans les conditions physiologiques.

9. Composé suivant la revendication 1 ou 2, dans lequel R représente un groupe (5-méthyl-1,3-dioxol-2-one-4-yl)méthyle, 1H-isobenzofuranne-3-one-1-yle, gamma-butyrolactone-4-yle, -CHR²OCOR³ ou -CHR²OCOOR³ dans lequel R² représente l'hydrogène ou un groupe méthyle et R³ représente un groupe alkyle en C₁ à C₆.

10. Composé répondant à la formule 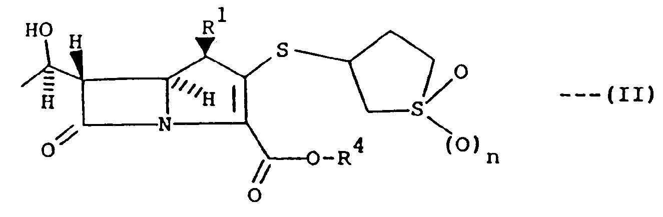 dans laquelle n est égal à 0 ou 1, R¹ représente l'hydrogène ou un groupe méthyle, et R⁴ représente un groupe protecteur classique d'acide carboxylique.

11. Composé suivant la revendication 10, dans lequel R⁴ représente un groupe benzyle, p-nitrobenzyle ou -CH₂CX=CH₂ dans lequel X représente H ou Cl.

12. Composé suivant la revendication 11, dans lequel R¹ représente l'hydrogène et R⁴ représente un groupe -CH₂CX=CH₂ dans lequel X représente l'hydrogène ou un groupe chloro.

13. Composition pharmaceutique comprenant un composé ou un de ses sels suivant l'une quelconque des revendications 1 à 9 et un diluant ou support pharmaceutiquement acceptable.

14. Composé ou un de ses sels suivant l'une quelconque des revendications 1 à 9, destiné à être utilisé comme médicament.

15. Utilisation d'un composé ou d'un de ses sels suivant l'une quelconque des revendications 1 à 9, pour la production d'un agent antibactérien.

## Revendications (Revendications pour l'(les) Etat(s) contractant(s) suivant(s): ES)

1. Procédé de préparation d'un composé répondant à la formule 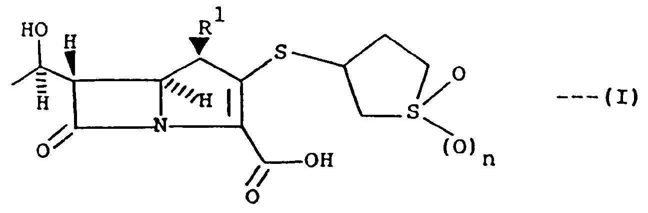 dans laquelle n est égal à 0 ou 1 ; et R¹ représente l'hydrogène ou un groupe méthyle ; ou d'un de ses sels cationiques pharmaceutiquement acceptables lorsque R représente l'hydrogène, qui comprend une élimination classique de la protection d'un composé répondant à la formule 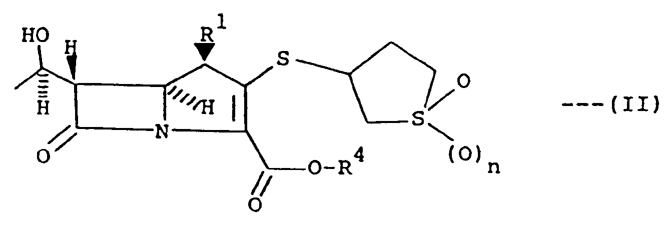 dans laquelle n est égal à 0 ou 1 ; R¹ représente l'hydrogène ou un groupe méthyle et R⁴ représente un groupe classique protecteur de l'acide carboxylique.

2. Procédé suivant la revendication 1, dans lequel R¹ représente l'hydrogène.

3. Procédé suivant la revendication 2, dans lequel n est égal à 1.

4. Procédé suivant la revendication 2, dans lequel n est égal à 0.

5. Procédé suivant la revendication 4, dans lequel les groupes fixés au noyau thiolane sont en position cis l'un par rapport à l'autre.

6. Procédé suivant la revendication 5, dans lequel les configurations sur le noyau thiolane sont les configurations 1R,3S, c'est-à-dire

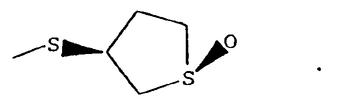

7. Procédé suivant la revendication 1 ou 2, dans lequel R⁴ représente un groupe -CH₂CX=CH₂ dans lequel X représente l'hydrogène ou un groupe chloro.

## Revendications (Revendications pour l'(les) Etat(s) contractant(s) suivant(s): GR)

1. Procédé de préparation d'un composé répondant à la formule 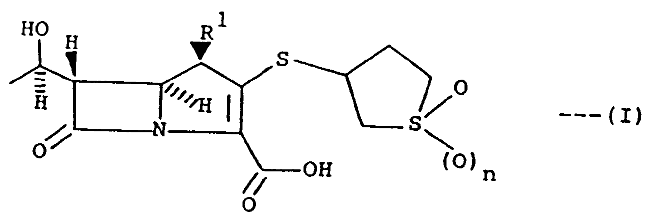 dans laquelle n est égal à 0 ou 1 ; et R¹ représente l'hydrogène ou un groupe méthyle ; ou d'un de ses sels cationiques pharmaceutiquement acceptables lorsque R représente l'hydrogène, qui comprend une élimination classique de la protection d'un composé répondant à la formule 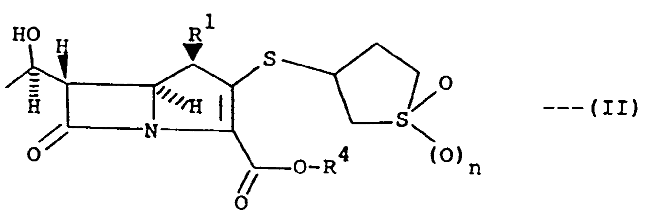 dans laquelle n est égal à 0 ou 1 ; R¹ représente l'hydrogène ou un groupe méthyle et R⁴ représente un groupe classique protecteur de l'acide carboxylique.

2. Procédé suivant la revendication 1, dans lequel R¹ représente l'hydrogène.

3. Procédé suivant la revendication 2, dans lequel n est égal à 1.

4. Procédé suivant la revendication 2, dans lequel n est égal à 0.

5. Procédé suivant la revendication 4, dans lequel les groupes fixés au noyau thiolane sont en position cis l'un par rapport à l'autre.

6. Procédé suivant la revendication 5, dans lequel les configurations sur le noyau thiolane sont les configurations 1R,3S, c'est-à-dire

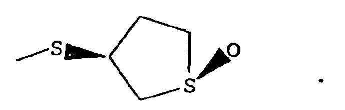

7. Procédé suivant la revendication 1 ou 2, dans lequel R⁴ représente un groupe -CH₂CX=CH₂ dans lequel X représente l'hydrogène ou un groupe chloro.

8. Composé répondant à la formule 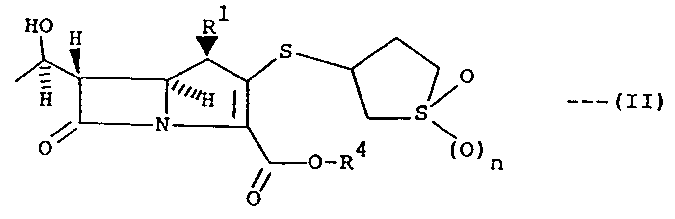 dans laquelle n est égal à 0 ou 1, R¹ représente l'hydrogène ou un groupe méthyle, et R⁴ représente un groupe classique protecteur de l'acide carboxylique.

9. Composé suivant la revendication 8, dans lequel R¹ représente l'hydrogène et R⁴ représente un groupe -CH₂CX=CH₂ dans lequel X représente l'hydrogène ou un groupe chloro.
